# EUROPEAN PATENT SPECIFICATION

(11) **EP 2 371 360 B1**
(45) Date of publication and mention of the grant of the patent: **29.11.2017**
(21) Application number: 11157781.3
(22) Date of filing: 11.03.2011
(51) Int. Cl.: A61K 9/70, A61K 47/14, A61K 31/137

(54) **Selegiline-containing adhesive preparation**
Selegilinhaltige Haftmittelherstellung
Préparation adhésive contenant de la sélégiline

(30) Priority: 12.03.2010 JP 2010056674
(43) Date of publication of application: 05.10.2011
(73) Proprietor: Fujimoto Co., Ltd., Matsubara-shi Osaka (JP)
(72) Inventor: Ameyama, Satoshi, Ibaraki-shi Osaka (JP); Nishiura, Eri, Ibaraki-shi Osaka (JP); Nakamura, Koji, Ibaraki-shi Osaka (JP); Kamiyama, Masashi, Ibaraki-shi Osaka (JP); Maruo, Hiroki, Ibaraki-shi Osaka (JP); Hosaka, Kyoko, Ibaraki-shi Osaka (JP); Hori, Mitsuhiko, Ibaraki-shi Osaka (JP)
(74) Representative: Grünecker Patent- und Rechtsanwälte PartG mbB

(56) References cited:
- EP-A1- 0 509 761
- EP-A1- 1 731 143
- EP-A1- 2 172 194
- EP-A2- 1 997 520
- EP-A2- 2 364 733
- EP-A2- 2 371 359
- WO-A1-00/59475
- WO-A1-2005/072705
- DATABASE WPI Section Ch, Week 200745 Thomson Scientific, London, GB; Class B05, AN 2007-459719 XP002671015, CHU W; LIU C; WANG G; WU L; XU J; YUAN S; ZHANG Y: "Selegiline and/or chlorhydric acid selegiline paste matrix and preparation method", -& CN 1 907 271 A (HARBIN CITY JIANDI PHARM TECHNOLOGY CO L) 7 February 2007 (2007-02-07)

## Description

### FIELD OF THE INVENTION

This invention relates to an adhesive preparation which comprises (-)-(R)-N,α-dimethyl-N-2-propynylphenethylamine (to be referred to as "free form of selegiline" hereinafter) and/or a pharmaceutically acceptable salt thereof (to be referred to as "salt of selegiline" hereinafter, and both of this salt and the aforementioned "free form of selegiline" to be referred inclusively to as "selegiline"). Specifically, the invention relates to an adhesive preparation which is attached onto skin surface to continuously administrating selegiline from skin surface into living body.

### BACKGROUND OF THE INVENTION

A basic drug, selegiline, is effective as an antiparkinsonism drug and is known as an inhibitor of monoamine oxidase (MAO). There are different subtypes of MAO, i.e., type A (MAO-A) and type B (MAO-B), and selegiline as an oral administration preparation is a selective inhibitor of type B. However, it has been reported so far that oral administration of selegiline in a large amount causes side effects such as reduction of the MAO-B selectivity as well as inhibition of MAO-A which is frequently distributed in the digestive tract. In addition, since metabolism of selegiline in the body is so rapid that it is difficult to maintain its concentration in blood at a desired level by oral administration.

When administered through an adhesive preparation, in general, absorbed amount (rate of absorption) of a drug depends on the drug concentration in the preparation, and the drug shifts into the skin by passive diffusion. Thus, it is known that the transition rate of the drug into the skin is lowered by reduction of the drug concentration in the preparation. However, when efficacy of the drug and its side effects are taken into consideration, it is necessary to reduce variation of the drug concentration in blood.

Based on the above, concern has been directed toward the development of an adhesive preparation which is capable of controlling reduction of the transition rate (permeation rate) of selegiline into the skin thereby keeping a constant rate.

In general, in order to effect absorption of a drug into the body at a constant rate, it is necessary to control drastic reduction of drug concentration in the drug-containing layer, and for that purpose, there are known a reservoir type adhesive preparation which has a drug release controlling film, a matrix type adhesive preparation that carries out release control of the drug by crystallization of the drug and the like, etc.

However, since the reservoir type depends on the film for its control, there is a case in which the effect thereof is considerably spoiled due to generation of a crack and the like during the production process, thereby causing release of the drug within a short period of time. Therefore, there is a possibility that this induces expression of toxicity of the drug. In addition, when the drug is liquid, it is difficult to effect the release control by crystallization of the drug.

In addition to the above-mentioned methods, there is a method in which reduction of the concentration of the drug is alleviated by increasing the drug content. As the method for increasing the drug content, there are a method in which thickness of the preparation is increased, a method in which the drug ratio in the composition is increased, and the like. However, the former is experientially known that peeling is apt to occur during wearing, and the latter has a problem of causing reduction of the pressure-sensitive adhesiveness.

As the release controlling method, in addition to the above methods, there is also a method in which diffusion rate of a drug in a preparation is lowered to thereby lower the release rate as a result. However, in this case, the permeation rate does not become constant but attenuation of the rate can be seen after showing the maximum permeation rate. Particularly, in the case of a liquid drug having high transition ratio into the skin, namely skin permeability, it becomes difficult to release the drug at a constant rate for a long time. Accordingly, there has been a demand for a substance which has an effect of inhibiting transition of the drug into the skin without depending on the drug release ability.

In this connection, although middle chain fatty acid triglyceride and diisopropyl adipate are known as skin permeation accelerators (see JP-A-2000-281570, International Publication WO 2006/082888 and JP-A-10-218793), it has not been known that they have an action to inhibit transition of drugs into the skin. Also, CN 1 907 271 A discloses a transdermal therapeutic system containing a patch substrate comprising selegiline, wherein triethyl citrate is used as a plasticizer, and eucalyptus oil or peppermint oil are used as transdermal permeation enhancers. In EP 1 731 143 A1, percutaneous absorption-type pharmaceutical preparations comprising selegiline (or selegiline hydrochloride) and a liquid plasticizer are disclosed. EP 0 509 761 A1 relates to salve or ointment compositions comprising deprenyl (selegiline hydrochloride) and Cremophor EL (polyethoxylated castor oil, having triglyceride as a main component). EP 2 172 194 A1 discloses a transdermal patch comprising an adhesive matrix layer which inter alia contains selegiline, an acrylic adhesive and triethyl citrate.

### SUMMARY OF THE INVENTION

The problem that the invention is to solve is to provide an adhesive preparation which enables administration of selegiline stably for a prolonged period of time.

The present inventors have conducted intensive studies and found as a result that when a specific liquid component is contained in a pressure-sensitive adhesive layer which contains selegiline, transition of selegiline into the skin is inhibited and transition rate (permeation rate) of selegiline into the skin can be stabilized for a long period of time, thereby resulting in the accomplished of the invention.

Namely, the present invention provides the following items.
1. Use of a component which is liquid at 25°C and has two or more ester bonds in one molecule thereof in an adhesive preparation comprising (-)-(R)-N,α-dimethyl-N-2-propynylphenethylamine and/or a pharmaceutically acceptable salt thereof, in order to inhibit the transition of (-)-(R)-N,α-dimethyl-N-2-propynylphenethylamine and/or a pharmaceutically acceptable salt thereof into the skin and to stabilize the transition rate of (-)-(R)-N,α-dimethyl-N-2-propynylphenethylamine and/or a pharmaceutically acceptable salt thereof into the skin;
   wherein the adhesive preparation comprises a backing and a pressure-sensitive adhesive layer formed on at least one side of the backing, the pressure-sensitive adhesive layer comprising (-)-(R)-N,α-dimethyl-N-2-propynylphenethylamine and/or a pharmaceutically acceptable salt thereof, a pressure-sensitive adhesive and a component which is liquid at 25°C and has two or more ester bonds in one molecule thereof; wherein the component which is liquid at 25°C is selected from the group consisting of: diisopropyl adipate and a middle chain fatty acid triglyceride, wherein the number of carbons in the middle chain fatty acid is from 8 to 12; and
   wherein the component which is liquid at 25°C is contained at a content of 15 to 50% by weight based on the total weight of the pressure-sensitive adhesive layer.
2. The use according to item 1, wherein the component which is liquid at 25°C is a middle chain fatty acid triglyceride, wherein the number of carbons in the middle chain fatty acid is from 8 to 12.
3. The use according to item 1, wherein the component which is liquid at 25°C is diisopropyl adipate.
4. The use according to item 1, wherein the content of (-)-(R)-N,α-dimethyl-N-2-propynylphenethylamine and/or a pharmaceutically acceptable salt thereof is within the range of from 1% by weight to 20% by weight based on the total weight of the pressure-sensitive adhesive layer, and wherein the content of pressure-sensitive adhesive is within the range of from 30% by weight to 80% by weight based on the total weight of the pressure-sensitive adhesive layer.
5. The use according to any one of items 1 to 4, wherein the pressure-sensitive adhesive is an acrylic pressure-sensitive adhesive.
6. The use according to any one of items 1 to 5, wherein the pharmaceutically acceptable salt of (-)-(R)-N,α-dimethyl-N-2-propynylphenethylamine is a hydrochloride.

According to the invention, there can be provided an adhesive preparation which is capable of administrating selegiline stably for a prolonged period of time and has fewer possibility of causing side effects.

### BRIEF DESCRIPTION OF THE DRAWINGS

Fig. 1 is a graph showing changes in the permeation rate of Examples 1 to 3 and Comparative Examples 1 to 3 into the skin of hairless mice.
Fig. 2 is a graph showing changes in the drug release rate of Examples 1 and 3 and Comparative Examples 1 and 2.

### DETAILED DESCRIPTION OF THE INVENTION

The following describes the invention in detail.

The adhesive preparation used in the invention is for effecting percutaneous absorption of selegiline, contains selegiline in its pressure-sensitive adhesive layer and can be used as an antiparkinsonism drug and an antidepressant. In addition, as its other applications, there may be mentioned an anti-Alzheimer disease agent, an antiepileptic, seasickness prevention, treatment of schizophrenia, maintenance and protection of nerve cell function, improvement of acetylcholine system neurotransmitter, treatment of glaucoma, prevention of senescence, treatment of HIV-related cognition function disorder, treatment of ADHD (attention-deficit hyperactivity disorder) and the like.

Selegiline as the active ingredient of the adhesive preparation used in the invention can be contained in the pressure-sensitive adhesive layer in a dissolved state, a dispersed state and/or a crystalline state.

As the pharmaceutically acceptable salt of selegiline, for example, there may be mentioned a salt with an inorganic acid, such as hydrochloride, hydrobromide, phosphate, nitrate, sulfate and the like, and a salt with an organic acid, such as acetate, oxalate, maleate, fumarate, tartrate, succinate and the like. Of these salts, hydrochloride (to be referred also to as "selegiline hydrochloride" hereinafter) is preferable from the viewpoint that when neutralized with a basic compound such as a metal hydroxide and the like, a metal chloride such as sodium chloride and the like, which inhibits reduction of cohesive strength and cohesive failure of the pressure-sensitive adhesive layer and thereby contributes to the stabilization of the preparation, can be formed.

Content of selegiline in the pressure-sensitive adhesive layer is within the range of from 0.5% by weight to 30% by weight, preferably from 1% by weight to 20% by weight, based on the total weight of the pressure-sensitive adhesive layer. When the content thereof is smaller than 0.5% by weight, there is a possibility that the desired therapeutic and preventive effects cannot be obtained, while when it is larger than 30% by weight, there is a possibility that a side effect due to high concentration selegiline is expressed.

As the backing to be used in the invention, although there is no particular limitation, a material in which contents of selegiline and liquid component described below are not reduced due to their loss from the backside through the backing, namely a material having impermeability for these components, is desirable. Illustratively, there may be mentioned a film made of a polyester such as polyethylene terephthalate, nylon, polyvinyl chloride, polyethylene, polypropylene, an ethylene-vinyl acetate copolymer, polytetrafluoroethylene, an ionomer resin and the like, a metal foil or a laminate film thereof and the like. Among them, in order to improve adhesiveness (anchoring property) with the pressure-sensitive adhesive layer, it is preferable to constitute the backing by a laminate film of a nonporous film made of the above-mentioned material with a porous film and form the pressure-sensitive adhesive layer on the porous film side.

The above-mentioned porous film is not particularly limited so long as the anchoring property of the pressure-sensitive adhesive layer is appropriate, and for example, there may be mentioned paper, woven fabric, non-woven fabric, a mechanically punching-treated sheet and the like, of which paper, woven fabric or non-woven fabric is particularly preferable. When improvement of the anchoring property and flexibility of the adhesive preparation are taken into consideration, thickness of such a porous film is generally from about 10 µm to about 500 µm, and in the case of a thin adhesive preparation such as a plaster type or pressure-sensitive adhesive tape type, it is generally from about 10 µm to about 200 µm. In addition, in the case of woven fabric and non-woven fabric, it is desirable to set their filling amount to a level of from 5 g/m² to 30 g/m² from the viewpoint of improving anchoring strength.

The pressure-sensitive adhesive layer used according to the invention is formed on at least one side of the backing. As the pressure-sensitive adhesive to be contained in the pressure-sensitive adhesive layer used according to the invention, an acrylic pressure-sensitive adhesive, a rubber-based pressure-sensitive adhesive, a silicone-based pressure-sensitive adhesive, a vinyl ester-based pressure-sensitive adhesive and the like can be mentioned. Particularly, an acrylic pressure-sensitive adhesive containing an acrylic polymer is desirable from the viewpoint of skin adhesiveness as the adhesive preparation.

In general, the acrylic pressure-sensitive adhesive used according to the invention is a polymer which comprises at least an alkyl ester of (meth)acrylic acid (to be also referred to as (meth)acrylic acid alkyl ester or alkyl (meth)acylate) as a monomer component, preferably a copolymer of an alkyl ester of (meth)acrylic acid with other monomer which is copolymerizable with the alkyl ester of (meth)acrylic acid (to be referred simply to as "other monomer" hereinafter), in which the main component is the alkyl ester of (meth)acrylic acid.

As the alkyl group of the (meth)acrylic acid alkyl ester, from the viewpoint of stickiness to the human skin, the number of carbon atoms is preferably 4 or more, particularly the number of carbon atoms is from 4 to 13, and it may be a straight chain or a branched chain. Illustratively, there may be mentioned butyl, isobutyl, sec-butyl, tert-butyl, pentyl, hexyl, heptyl, n-octyl, iso-octyl, sec-octyl, tert-octyl, 2-ethylhexyl, nonyl, decyl, undecyl, dodecyl, tridecyl and the like, of which 2-ethylhexyl of preferred. The (meth)acrylic acid alkyl ester can be used alone or by a combination of two or more species.

As the other monomer, examples thereof include carboxyl group-containing monomers such as (meth)acrylic acid, itaconic acid, maleic acid, maleic anhydride and the like; sulfoxyl group-containing monomers such as styrene sulfonate, allyl sulfonate, sulfopropyl (meth)acrylate, (meth)acryloyloxynaphthalene sulfonate, acrylamidomethyl sulfonate and the like; hydroxyl group-containing monomers such as hydroxyethyl (meth)acrylate, hydroxypropyl(meth)acrylate; (meth)acrylic acid derivatives having amido group such as (meth)acrylamide, dimethyl (meth)acrylamide, hydroxyethyl (meth)acrylamide, N-butyl (meth)acrylamide, N-methylol (meth)acrylamide and the like; aminoalkyl esters of (meth)acrylic acid such as aminoethyl (meth)acrylate, dimethylaminoethyl (meth)acrylate, tert-butylaminoethyl (meth)acrylate and the like; alkoxy esters of (meth)acrylic acid such as methoxyethyl (meth)acrylate, ethoxyethyl (meth)acrylate, tetrahydrofurfuryl (meth)acrylate and the like; alkoxyalkylene glycol esters of (meth)acrylic acid such as methoxyethylene glycol (meth)acrylate, methoxydiethylene glycol (meth)acrylate, methoxypolyethylene glycol (meth)acrylate, methoxypolypropylene glycol (meth)acrylate and the like; (meth)acrylonitrile; compounds having vinyl group such as vinyl acetate, vinyl propionate, N-vinyl-2-pyrrolidone, methylvinylpyrrolidone, vinylpyridine, vinylpiperidone, vinylpyrimidine, vinylpiperazine, vinylpyrrole, vinylimidazole, vinylcaprolactam, vinyloxazole, vinylmorpholine and the like, and these may be used alone or as a combination of two or more species. Particularly, carboxyl group-containing monomers (preferably acrylic acid), hydroxyl group-containing monomers (preferably 2-hydroxyethyl acrylate), (meth)acrylic acid derivatives having amido group (preferably hydroxyethyl (meth)acrylamide), N-vinyl-2-pyrrolidone, vinyl acetate and the like are desirable from the viewpoint of pressure-sensitive adhesive characteristics.

Copolymerization ratio of the alkyl ester of (meth)acrylic acid and other monomer is not particularly limited and is arbitrarily set in response to the molecular weight characteristics of the copolymer to be obtained, such as weight average molecular weight and the like. Particularly preferable is a copolymer obtained by blending the alkyl ester of (meth)acrylic acid and other monomer at a weight ratio of alkyl ester of (meth)acrylic acid/other monomer = generally 50 to 97/50 to 3, preferably 65 to 95/35 to 5, followed by copolymerization.

As a desirable copolymer, for example, there may be mentioned a copolymer of 2-ethylhexyl acrylate, N-vinyl-2-pyrrolidone and acrylic acid; a copolymer of 2-ethylhexyl acrylate, 2-hydroxyethyl acrylate and vinyl acetate; a copolymer of 2-ethylhexyl acrylate and acrylic acid, and the like. From the viewpoint of pressure-sensitive adhesive characteristics of the copolymer, more preferred is a copolymer of 2-ethylhexyl acrylate, N-vinyl-2-pyrrolidone and acrylic acid, and particularly preferred is a copolymer obtained by blending 2-ethylhexyl acrylate, N-vinyl-2-pyrrolidone and acrylic acid at a weight ratio of 2-ethylhexyl acrylate/N-vinyl-2-pyrrolidone/acrylic acid = 50 to 90/10 to 30/0 to 5, followed by copolymerization.

Content of the pressure-sensitive adhesive in the pressure-sensitive adhesive layer is within the range of generally from 20% by weight to 90% by weight, preferably from 30% by weight to 80% by weight, based on the total weight of the pressure-sensitive adhesive layer. When the content thereof is smaller than 20% by weight, there is a possibility that it is difficult to maintain skin adhesive strength of the adhesive preparation, while when it is larger than 90% by weight, there is a possibility of generating a skin irritation due to strong skin adhesive strength.

The adhesive preparation of the invention contains, in the pressure-sensitive adhesive layer, a liquid component having two or more ester bonds in one molecule thereof. Such a liquid component is a component which is liquid at 25°C, and from the viewpoint of securing desired physical properties, the liquid component is a middle chain fatty acid triglyceride, and the middle chain fatty acid triglyceride is a triglyceride in which at least one of the three fatty acids bonding to glycerol by an ester bond is a middle chain fatty acid (the number of carbons therein is from 8 to 12), more preferred is a triglyceride in which at least two of the three fatty acids bonding to glycerol by an ester bond are a middle chain fatty acid (the number of carbons therein is from 8 to 12), and most preferred is a triglyceride in which all of the three fatty acids bonding to glycerol by an ester bond are a middle chain fatty acid (the number of carbons therein is from 8 to 12).

Also, in the middle chain fatty acid triglyceride, a triglyceride in which the middle chain fatty acid species (in which the number of carbons is from 8 to 12) that bonds to glycerol by an ester bond is only one species (e.g., caprylic acid triglyceride in which the middle chain fatty acid bonding to glycerol by an ester bond is caprylic acid alone, capric acid triglyceride in which the middle chain fatty acid bonding to glycerol by an ester bond is capric acid alone, and the like) may be used, or a triglyceride in which the middle chain fatty acid species (in which the number of carbons is from 8 to 12) that bonds to glycerol by an ester bond are two or more species (e.g., (caprylic acid/capric acid) triglyceride in which the middle chain fatty acids that bond to glycerol by an ester bond are caprylic acid and capric acid, (caprylic acid/capric acid/lauric acid) triglyceride in which the middle chain fatty acids that bond to glycerol by an ester bond are caprylic acid, capric acid and lauric acid, and the like) may be used. As the middle chain fatty acid triglyceride in the invention, one species of middle chain fatty acid triglyceride alone may be used or a mixture of two or more species of middle chain fatty acid triglyceride may be used.

In addition, the middle chain fatty acid triglyceride may be an extract from a natural material or a synthesized product. In addition, a commercial item can also be used, and for example, there may be mentioned "COCONARD" manufactured by Kao Corp., "Crodamol GTCC" manufactured by Croda Inc., "PANACET 810S" manufactured by NOF CORPORATION and the like.

Alternatively, the liquid component is diisopropyl adipate.

Content of the liquid component is 15% by weight or more, based on the total weight of the pressure-sensitive adhesive layer. In addition, the upper limit of the content of the liquid component is 50% by weight or less.

According to the adhesive preparation used in the invention, the pressure-sensitive adhesive layer may be non-crosslinked, but in the case of preventing excess plasticization, a crosslinking treatment may be applied. In that case, as the crosslinking agent for applying a crosslinking treatment to the pressure-sensitive adhesive layer, for example, there may be mentioned an organic metal compound, a metal alcoholate, a metal chelate compound and the like. Illustratively, examples of the organic metal compound include zirconium, zinc alaninate, zinc acetate, glycine ammonium zinc and the like. Examples of the metal alcoholate include tetraethyl titanate, tetraisopropyl titanate, aluminum isopropylate, aluminum sec-butylate and the like. Examples of the metal chelate compound include di-iso-propoxybis(acetylacetone) titanate, tetraoctylene glycol titanate, aluminum isopropylate, ethyl acetoacetate aluminum diisopropylate, aluminum tris(ethyl acetoacetate), aluminum tris(acetyl acetonate) and the like. In this connection, a suitably used crosslinking agent is a metal chelate compound. Particularly, ethyl acetoacetate aluminum diisopropylate is more preferable. For the crosslinking treatment, the above-mentioned crosslinking agents may be used alone or as a combination of two or more species.

In the case of applying a crosslinking treatment to the pressure-sensitive adhesive layer, content of the crosslinking agent varies depending on the kinds of the crosslinking agent and pressure-sensitive adhesive but is generally within the range of from 0.05 part by weight to 0.6 part by weight, preferably from 0.12 part by weight to 0.4 part by weight, based on 100 parts by weight of the pressure-sensitive adhesive layer to which the crosslinking treatment is applied.

In order to increase absorption of selegiline, a metal hydroxide may be contained in the pressure-sensitive adhesive layer of the adhesive preparation of the invention. As the metal hydroxide, for example, sodium hydroxide, calcium hydroxide, magnesium hydroxide and the like may be mentioned, of which sodium hydroxide is desirable.

From the viewpoint of applying the adhesive preparation to the skin and peeling therefrom, thickness of the pressure-sensitive adhesive layer is generally from 10 µm to 300 µm, preferably from 50 µm to 200 µm.

According to the necessity, the pressure-sensitive adhesive layer may be blended with an additive agent such as various pigments, various fillers, a stabilizer, a drug solubilizing agent, a drug solubilization inhibitor, a metal chloride and the like.

From the viewpoint of adhesion to skin, the pressure-sensitive adhesive layer is preferably a hydrophobic pressure-sensitive adhesive layer and more preferably a non-hydroscopic pressure-sensitive adhesive layer. The term "non-hydroscopic pressure-sensitive adhesive layer" as used herein is not always limited to those which are completely free from moisture, but those which contain a slight amount of moisture derived from the air humidity, the skin and the like are included therein. The term "a slight amount of moisture" as used herein is, as the moisture content of the layered product of backing and pressure-sensitive adhesive layer, preferably 5% by weight or less, more preferably 2% by weight or less, most preferably 1% by weight or less. In this case, the moisture content of the layered product of backing and pressure-sensitive adhesive layer means weight ratio of water contained in the layered product of backing and pressure-sensitive adhesive layer after separating a release liner when present (i.e., weight percentage of water based on the total weight of the layered product of backing and pressure-sensitive adhesive layer) which is measured by the coulometric Karl Fischer titration method, and is illustratively as follows. That is, under an environment controlled at a temperature of 23 ± 2°C and a relative humidity of 40 ± 5% RH, a test piece is prepared by punching a sample having a release liner when present, into a predetermined size. Then, after peeling off the release liner when present, the resulting test piece is put into a moisture vaporizer. The test piece is heated at 140°C in the moisture vaporizer, the moisture generated is then introduced into a titration flask using nitrogen as the carrier, and the moisture content (% by weight) of the sample is measured by the coulometric Karl Fischer titration method.

The production method of the adhesive preparation used according to the invention is not particularly limited, but for example, it can be produced by the following production method.

Firstly, a drug-containing solution containing free form of selegiline and/or a salt of selegiline is prepared using a solvent such as ethanol.

In this connection, in the case of containing a metal hydroxide in the pressure-sensitive adhesive layer, the drug-containing solution is prepared by mixing and stirring free form of selegiline and/or a salt of selegiline with a metal hydroxide dissolved and/or dispersed in a solvent such as ethanol.

The above-mentioned drug-containing solution is dissolved or dispersed in a solvent or dispersion medium together with a pressure-sensitive adhesive (e.g., an acrylic copolymer pressure-sensitive adhesive and the like), a liquid component, and, in response to the necessity, a crosslinking agent, other additives and the like. In this connection, since the salt of selegiline has low solubility for the pressure-sensitive adhesive layer, there is a tendency to form a dispersed state. The solvent or dispersion medium to be used in forming the pressure-sensitive adhesive layer is not particularly limited, and those which are generally used as a solvent and the like for a pressure-sensitive adhesive can be selected by taking kind of the pressure-sensitive adhesive, its reactivity with the drug, and the like into consideration. As such a solvent or dispersion medium, ethyl acetate, toluene, hexane, 2-propanol, methanol, ethanol and the like may for example be mentioned.

Next, a pressure-sensitive adhesive layer is formed by coating the thus obtained solution or dispersion on one side of the backing or the release treatment side of a release sheet, followed by drying. In this connection, it is possible to carry out the aforementioned coating by, for example, a technique conventionally known to those skilled in the art, such as casting, printing and the like. Thereafter, the release sheet or backing is pasted to the pressure-sensitive adhesive layer. As such a release sheet, there is no particular limitation so long as it can be easily peeled off from the pressure-sensitive adhesive layer when used, and for example, there may be used a film such as of polyester, polyvinyl chloride, polyvinylidene chloride, polyethylene terephthalate and the like in which a silicone treatment was applied to its contacting side with the pressure-sensitive adhesive layer, or a laminated film of wood free paper or glassine paper with polyolefin, and the like. Thickness of the release sheet is generally 200 µm or less, preferably from 25µm to 100 µm. In this connection, when a crosslinking treatment is carried out, the adhesive preparation of the invention is prepared by, after pasting the release sheet to the pressure-sensitive adhesive layer, accelerating the crosslinking reaction by applying an aging treatment and the like at generally from 60°C to 90°C, preferably from 60°C to 70°C, for a period of from 24 hours to 48 hours.

In this connection, the adhesive preparation may also be formed as follows. Namely, after preparing a drug-containing solution by dissolving or dispersing selegiline in a solvent or dispersion medium together with a pressure-sensitive adhesive (e.g., an acrylic copolymer pressure-sensitive adhesive and the like), a liquid component, and, in response to the necessity, a crosslinking agent, other additives and the like, the thus obtained solution is subsequently mixed with a basic compound and/or a metal chloride while stirring. Thereafter, the solution is then coated on one side of the backing or the release treatment side of a release sheet, followed by drying, thereby forming a pressure-sensitive adhesive layer. Then, the pressure-sensitive adhesive layer is pasted to the release sheet or backing.

Shape of the adhesive preparation of the invention is not limited, and for example, it may be a tape shape, a sheet shape and the like.

Dose of the adhesive preparation of the invention varies depending on the age, body weight, symptoms and the like of each patient, but it is desirable to apply an adhesive preparation containing from 1 mg to 40 mg of selegiline, generally to the skin of an adult within an area of from 1 cm² to 40 cm² approximately from once per two days to twice a day.

### Examples

The following describes the invention in detail with reference to examples. In this connection, the "part(s)" and "%" as used in the following descriptions mean "part(s) by weight" and "%" by weight, respectively.

### (Preparation of acrylic pressure-sensitive adhesive)

Under an inert gas atmosphere, 72 parts of 2-ethylhexyl acrylate (2-EHA), 25 parts of N-vinyl-2-pyrrolidone (VP), 3 parts of acrylic acid (AA) and 0.2 part of azobisisobutyronitrile were allowed to undergo solution polymerization in ethyl acetate at 60°C, thereby preparing a solution of an acrylic pressure-sensitive adhesive. The weight average molecular weight of the acrylic pressure-sensitive adhesive was about 1,800,000.

### (Preparation of selegiline-containing adhesive preparations of Examples 1 to 3 and Comparative Examples 1 to 3)

Each pressure-sensitive adhesive solution was prepared in accordance with the blending ratio shown in the following Table 1, and its viscosity was adjusted with ethyl acetate. The thus obtained solution was coated on a polyester film (75 µm in thickness) so that the thickness of a pressure-sensitive adhesive layer after drying became 80 µm and then dried to prepare the pressure-sensitive adhesive layer. Subsequently, this pressure-sensitive adhesive layer was pasted on a polyester film (12 µm in thickness) and then an aging treatment was carried out at 60°C for 48 hours, thereby preparing a selegiline-containing adhesive preparation.

In this connection, "COCONARD MT" ((caprylic acid/capric acid) triglyceride, mfd. by Kao Corp.) was used as the middle chain fatty acid triglyceride. In addition, in the Table 1, ALCH represents ethyl acetoacetate aluminum diisopropylate, and the all the units are "% by weight" based on the total weight of the pressure-sensitive adhesive layer.

**Table 1**

| | Liquid component | | | Selegiline hydrochloride | Others | |
|---|---|---|---|---|---|---|
| | Name | Molecular weight | Content (% by weight) | Content (% by weight) | Name | Content (% by weight) |
| Example 1 | Middle chain fatty acid triglyceride | 453-537 | 40 | 9 | Sodium hydroxide | 1.61 |
| | | | | | Pressure-sensitive adhesive | 49.24 |
| | | | | | ALCH | 0.15 |
| Example 2 Example 2 | Middle chain fatty acid triglyceride | 453-537 | 20 | 9 | Sodium hydroxide | 1.61 |
| | | | | | Pressure-sensitive adhesive | 69.39 |
| Reference Example 3 | Diisopropyl adipate | 230 | 40 | 9 | Sodium hydroxide | 1.61 |
| | | | | | Pressure-sensitive adhesive | 49.24 |
| | | | | | ALCH | 0.15 |
| Comparative Example 1 | Isopropyl myristate | 270 | 40 | 9 | Sodium hydroxide | 1.61 |
| | | | | | Pressure-sensitive adhesive | 49.24 |
| | | | | | ALCH | 0.15 |
| Comparative Example 2 | - | - | - | 9 | Sodium hydroxide | 1.61 |
| | | | | | Pressure-sensitive adhesive | 89.39 |
| Comparative Example 3 | Hexyl laurate | 284 | 40 | 9 | Sodium hydroxide | 1.61 |
| | | | | | Pressure-sensitive adhesive | 49.24 |
| | | | | | ALCH | 0.15 |

### (Evaluation tests)

### Skin permeation test (hairless mouse-extracted skin)

Skin permeation test was carried out on the adhesive preparations of Examples 1, 2, Reference Example 3, and Comparative Examples 1 to 3. Method of the skin permeation test is as follows.

Each preparation (sample) was applied to the center of a hairless mouse-extracted skin which had been thoroughly hydrated and punched into a size of 2.54 cm², and set to a permeation cell. The test was started by passing a receptor liquid kept at 32°C and activating a fraction collector. The receptor liquid was recovered after 4, 8, 12, 16, 20 or 24 hours. Content of selegiline in the recovered receptor liquid was determined using a high performance liquid chromatography (HPLC) and the permeation rate (flux, amount of permeated drug per unit time (hr) unit area (cm²), (µg/cm²/hr), was calculated.

The test conditions and HPLC assay conditions are as follows.

### (Test conditions)

Permeation apparatus: a full automatic flow through diffusion cell apparatus (mfd. by Vanguard International)
Sample area: 0.5 cm²
Receptor liquid: phosphate buffer (pH = 7.4, containing 0.02% by weight of sodium azide)
Flow rate: about 10 ml/4 hr/cell

### (HPLC assay conditions)

Detector: an ultraviolet absorptiometer (wavelength; 205 nm)
Column: ODS-3 manufactured by GL Science Inc.
Column temperature: 35°C
Mobile phase: An 11.50 g portion of ammonium dihydrogenphosphate was dissolved in 1000 ml of water and adjusted to pH 3.1 with phosphoric acid. To 900 ml of this liquid, a 100 ml portion of acetonitrile for liquid chromatography was added and mixed.
Flow rate: About 1.4 ml/min

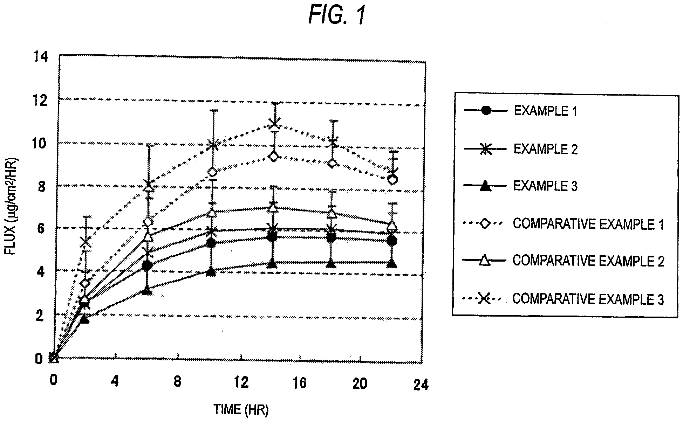

The results are shown in Fig. 1.

### <Dissolution test>

A dissolution test was carried out on the adhesive preparations of Examples 1, 2, Reference Example 3 and Comparative Examples 1 and 2. Method of the skin permeation test is as follows.

A 1000 ml portion of the No. 2 solution of dissolution test of the Pharmacopoeia of Japan was kept at 32°C and used as the test liquid, and the test was carried out by the rotary cylinder method of the dissolution test method (USP 30 <724> Drug Release Apparatus 6) at 50 rotations per minute. The test liquid was recovered in 5 ml portions after 10, 20, 30, 40, 60, 90, 120 and 240 minutes. (After each recovery, 5 ml of the test liquid was added thereto.) The selegiline content of the recovered test liquid was determined using the HPLC assay method. In this connection, a dissolution tester NTR-8000AC (TOYAMA SANGYO CO., LTD.) was used as the test apparatus, and the aforementioned conditions were used for the HPLC assay.

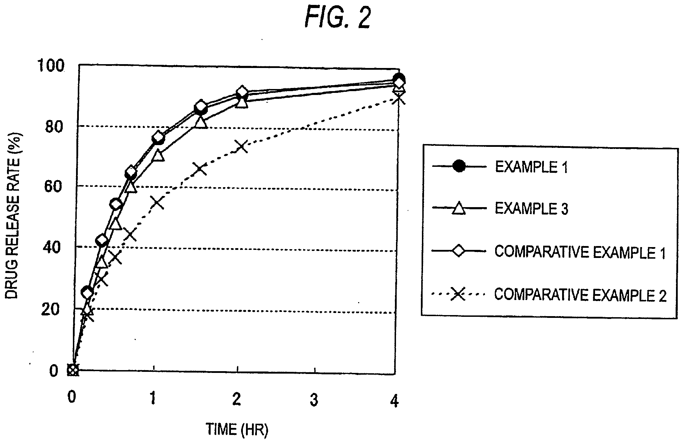

The results are shown in Fig. 2.

Based on the skin permeation test (Fig. 1), in the case of the adhesive preparations blended with an acrylic pressure-sensitive adhesive, which were further blended with 40% of a middle chain fatty acid triglyceride and diisopropyl adipate (Example 1 and Reference Example 3), the permeation rate was controlled at a lower level than that of the adhesive preparation which does not contain liquid component (Comparative Example 2), and reduction of the permeation rate after 16 hours of transition was not also found. It was unexpected that the permeation rate was controlled in the adhesive preparations containing liquid component, at a level lower than that of the adhesive preparation which does not contain the liquid component. In addition, in the adhesive preparations in which isopropyl myristate or hexyl laurate was blended as the liquid component in place of the middle chain fatty acid triglyceride or diisopropyl adipate (Comparative Examples 1 and 3), isopropyl myristate and hexyl laurate were acted as a permeation accelerator so that increase of the maximum permeation rate and reduction of the permeation rate after 16 hours of transition were observed.

On the other hand, in the dissolution test (Fig. 2), no difference was found between the middle chain fatty acid triglyceride, diisopropyl adipate and isopropyl myristate in the 40%-blended adhesive preparations. Only the adhesive preparations containing no liquid component showed low drug release property. Based on such results, although no difference can be found regarding the drug release property from the adhesive preparations, it was considered that the liquid component acts upon the skin and thereby inhibits drug transition and drug permeation into the skin. In addition, in the 20% middle chain fatty acid triglyceride-blended adhesive preparation (Example 2), the same effect as the case of 40% blending (Example 1) was found.

## Claims

1. Use of a component which is liquid at 25°C and has two or more ester bonds in one molecule thereof in an adhesive preparation comprising (-)-(R)-N,α-dimethyl-N-2-propynylphenethylamine and/or a pharmaceutically acceptable salt thereof, in order to inhibit the transition of (-)-(R)-N,α-dimethyl-N-2-propynylphenethylamine and/or a pharmaceutically acceptable salt thereof into the skin and to stabilize the transition rate of (-)-(R)-N,α-dimethyl-N-2-propynylphenethylamine and/or a pharmaceutically acceptable salt thereof into the skin;
wherein the adhesive preparation comprises a backing and a pressure-sensitive adhesive layer formed on at least one side of the backing, the pressure-sensitive adhesive layer comprising (-)-(R)-N,α-dimethyl-N-2-propynylphenethylamine and/or a pharmaceutically acceptable salt thereof, a pressure-sensitive adhesive and a component which is liquid at 25°C and has two or more ester bonds in one molecule thereof;
wherein the component which is liquid at 25°C is selected from the group consisting of: diisopropyl adipate and a middle chain fatty acid triglyceride, wherein the number of carbons in the middle chain fatty acid is from 8 to 12; and
wherein the component which is liquid at 25°C is contained at a content of 15 to 50% by weight based on the total weight of the pressure-sensitive adhesive layer.

2. The use according to claim 1, wherein the component which is liquid at 25°C is a middle chain fatty acid triglyceride, wherein the number of carbons in the middle chain fatty acid is from 8 to 12.

3. The use according to claim 1, wherein the component which is liquid at 25°C is diisopropyl adipate.

4. The use according to claim 1, wherein the content of (-)-(R)-N,α-dimethyl-N-2-propynylphenethylamine and/or a pharmaceutically acceptable salt thereof is within the range of from 1% by weight to 20% by weight based on the total weight of the pressure-sensitive adhesive layer, and wherein the content of pressure-sensitive adhesive is within the range of from 30% by weight to 80% by weight based on the total weight of the pressure-sensitive adhesive layer.

5. The use according to any one of claims 1 to 4, wherein the pressure-sensitive adhesive is an acrylic pressure-sensitive adhesive.

6. The use according to any one of claims 1 to 5, wherein the pharmaceutically acceptable salt of (-)-(R)-N,α-dimethyl-N-2-propynylphenethylamine is a hydrochloride.

## Patentansprüche

1. Verwendung einer Komponente, welche bei 25 °C flüssig ist und zwei oder mehr Esterbindungen in einem Molekül davon aufweist, in einer Klebstoffzubereitung, umfassend (-)-(R)-N,α-Dimethyl-N-2-propinylphenethylamin und/oder ein pharmazeutisch verträgliches Salz davon, um den Übergang von (-)-(R)-N,α-Dimethyl-N-2-propinylphenethylamin und/oder einem pharmazeutisch verträglichen Salz davon in die Haut zu hemmen und die Übergangsgeschwindigkeit von (-)-(R)-N,α-Dimethyl-N-2-propinylphenethylamin und/oder einem pharmazeutisch verträglichen Salz davon in die Haut zu stabilisieren;
wobei die Klebstoffzubereitung einen Träger und eine Haftklebstoffschicht, die auf wenigstens einer Seite des Trägers gebildet ist, umfasst, wobei die Haftklebstoffschicht (-)-(R)-N,α-Dimethyl-N-2-propinylphenethylamin und/oder ein pharmazeutisch verträgliches Salz davon, einen Haftklebstoff und eine Komponente, welche bei 25 °C flüssig ist und zwei oder mehr Esterbindungen in einem Molekül davon aufweist, umfasst;
wobei die Komponente, welche bei 25 °C flüssig ist, ausgewählt ist aus der Gruppe bestehend aus Diisopropyladipat und einem mittelkettigen Fettsäuretriglycerid, wobei die Anzahl der Kohlenstoffatome in der mittelkettigen Fettsäure 8 bis 12 beträgt; und
wobei die Komponente, welche bei 25 °C flüssig ist, in einem Gehalt von 15 bis 50 Gew.-%, bezogen auf das Gesamtgewicht der Haftklebstoffschicht, enthalten ist.

2. Verwendung gemäß Anspruch 1, wobei die Komponente, welche bei 25 °C flüssig ist, ein mittelkettiges Fettsäuretriglycerid ist, wobei die Anzahl der Kohlenstoffatome in der mittelkettigen Fettsäure 8 bis 12 beträgt.

3. Verwendung gemäß Anspruch 1, wobei die Komponente, welche bei 25 °C flüssig ist, Diisopropyladipat ist.

4. Verwendung gemäß Anspruch 1, wobei der Gehalt von (-)-(R)-N,α-Dimethyl-N-2-propinylphenethylamin und/oder einem pharmazeutisch verträglichen Salz davon in dem Bereich von 1 Gew.-% bis 20 Gew.-%, bezogen auf das Gesamtgewicht der Haftklebstoffschicht, liegt, und wobei der Gehalt des Haftklebstoffs in dem Bereich von 30 Gew.-% bis 80 Gew.-%, bezogen auf das Gesamtgewicht der Haftklebstoffschicht, liegt.

5. Verwendung gemäß einem der Ansprüche 1 bis 4, wobei der Haftklebstoff ein Acrylhaftklebstoff ist.

6. Verwendung gemäß einem der Ansprüche 1 bis 5, wobei das pharmazeutisch verträgliche Salz von (-)-(R)-N,α-Dimethyl-N-2-propinylphenethylamin ein Hydrochlorid ist.

## Revendications

1. Utilisation d'un composant qui est liquide à 25 °C et qui comporte deux ou plus de deux liaisons ester dans une molécule de celui-ci dans une préparation adhésive comprenant de la (-)-(R)-N,α-diméthyl-N-2-propynylphénéthylamine et/ou un sel pharmaceutiquement acceptable de celle-ci, afin d'inhiber la transition de (-)-(R)-N,α-diméthyl-N-2-propynylphénéthylamine et/ou d'un sel pharmaceutiquement acceptable de celle-ci dans la peau et de stabiliser la vitesse de transition de (-)-(R)-N,α-diméthyl-N-2-propynylphénéthylamine et/ou d'un sel pharmaceutiquement acceptable de celle-ci dans la peau ;
dans laquelle la préparation adhésive comprend un support et une couche adhésive sensible à la pression formée sur au moins une face du support, la couche adhésive sensible à la pression comprenant la (-)-(R)-N,α-diméthyl-N-2-propynylphénéthylamine et/ou un sel pharmaceutiquement acceptable de celle-ci, un adhésif sensible à la pression et un composant qui est liquide à 25 °C et qui comporte deux ou plus de deux liaisons ester dans une molécule de celui-ci ;
dans laquelle le composant qui est liquide à 25 °C est choisi dans le groupe consistant en : l'adipate de diisopropyle et un triglycéride d'acide gras à chaîne moyenne, le nombre de carbone dans l'acide gras à chaîne moyenne étant de 8 à 12 ; et
dans laquelle le composant qui est liquide à 25 °C est contenu à hauteur de 15 à 50 % en poids par rapport au poids total de la couche adhésive sensible à la pression.

2. Utilisation selon la revendication 1, dans laquelle le composant qui est liquide à 25 °C est un triglycéride d'acide gras à chaîne moyenne, dans laquelle le nombre de carbone dans l'acide gras à chaîne moyenne est de 8 à 12.

3. Utilisation selon la revendication 1, dans laquelle le composant qui est liquide à 25 °C est l'adipate de diisopropyle.

4. Utilisation selon la revendication 1, dans laquelle la teneur de (-)-(R)-N,α-diméthyl-N-2-propynylphénéthylamine et/ou d'un sel pharmaceutiquement acceptable de celle-ci est dans la plage de 1 % en poids à 20 % en poids par rapport au poids total de la couche adhésive sensible à la pression, et dans laquelle la teneur en adhésif sensible à la pression est dans la plage de 30 % en poids à 80 % en poids par rapport au poids total de la couche adhésive sensible à la pression.

5. Utilisation selon l'une quelconque des revendications 1 à 4, dans laquelle l'adhésif sensible à la pression est un adhésif sensible à la pression acrylique.

6. Utilisation selon l'une quelconque des revendications 1 à 5, dans laquelle le sel pharmaceutiquement acceptable de la (-)-(R)-N,α-diméthyl-N-2-propynylphénéthylamine est un chlorhydrate.
